# EUROPEAN PATENT APPLICATION

(11) **EP 4 600 340 A1**
(43) Date of publication of application: **13.08.2025**
(21) Application number: 22961242.9
(22) Date of filing: 08.10.2022
(51) Int. Cl.: C12M 1/00, C12M 1/36, C12Q 1/6869

(54) **FLUID SYSTEM, FLUID TRANSPORTATION METHOD, GENE SEQUENCER, AND BIOCHEMICAL DETECTION METHOD**

(71) Applicant: MGI Tech Co., Ltd., Shenzhen, Guangdong 518083 (CN)
(72) Inventor: NIU, Zihua, Shenzhen, Guangdong 518083 (CN); LU, Hao, Shenzhen, Guangdong 518083 (CN); XING, Chutian, Shenzhen, Guangdong 518083 (CN); CUI, Xingye, Shenzhen, Guangdong 518083 (CN)
(74) Representative: Haseltine Lake Kempner LLP
(86) International application number: PCT/CN2022/123789
(87) International publication number: WO 2024/073885

(57) **Abstract**

Provided are a fluid system, a fluid transport method, a gene sequencer, and a biochemical assay method. The fluid transport method includes the following steps: step a) of aspirating a first fluid from a fluid cartridge by using a pump valve assembly, so that the first fluid is input from an outlet (141) of a flow cell (115) into a lane (107) of the flow cell (115), and the first fluid output from an inlet (142) of the flow cell (115) is returned to the fluid cartridge; and/or step b) of aspirating a second fluid from a fluid cartridge by using a pump valve assembly, so that the second fluid is input from the inlet (142) of the flow cell (115) into the lane (107) of the flow cell (115), and the second fluid output from the outlet (141) of the flow cell (115) is returned to the fluid cartridge. The sample or reagent is reversely transported from the outlet (141) to the inlet (142) of the flow cell (115), and the reversely loaded sample or reagent may be directly returned to a sample cartridge (113) or a reagent kit finally, so that the sequencing time may be shortened, the sample/reagent loading flexibility may be improved, and the sequencing efficiency may be increased.

## Description

### TECHNICAL FIELD

The present disclosure relates to a fluid system, a fluid transport method, a gene sequencer, and a biochemical assay method.

### BACKGROUND

After decades of development, gene sequencing technology has been widely used in the field of medical health, such as infectious disease tracing, targeted tumor treatment, and whole genome testing. These diverse applications require gene sequencers to have increasing flexibility, especially in terms of types and quantities of samples supported in each sequencing run. To meet this demand, a gene sequencing chip is generally designed to have a plurality of lanes each loaded with different DNA samples. On the other hand, reagents used in a gene sequencing process are expensive, and an amount of reagents needs to be minimized. Therefore, there is typically only one pipeline connecting the sequencing chip and the reagent kit to reduce a pipeline volume and a loss of reagents in the pipeline. The pipeline may be divided into a plurality of branches at a front end of the sequencing chip, to connect to the plurality of lanes of the chip respectively. This leads to a contradiction that different samples are inevitably mixed in the pipeline before reaching different lanes of the chip, thus failing to meet the requirement that each lane corresponds to one sample.

To achieve loading of multiple samples, three methods are generally adopted in the prior art.

A first method is to pre-treat different DNA samples by attaching a known base sequence as a "barcode" to distinguish samples. The samples are then mixed together and uniformly loaded into the lanes of the chip. After sequencing is completed, the mixed samples may be split and identified using the barcode.

A second method is to load samples outside the sequencer (off-sequencer), which requires a specially designed automated instrument or is implemented manually. The samples are firstly loaded into the chip, and then the chip is transferred to the sequencer for subsequent sequencing steps.

A third method is to load samples in reverse through a fluid system on the sequencer so that different samples are loaded into different chip lanes.

These three existing methods described above have the following disadvantages.

In the first method, the samples are mixed by means of barcode, and the mixed samples need to be split and identified using the barcode at the end of sequencing, which greatly increases sequencing cost and sequencing time.

In the second method, loading samples outside the sequencer involves many steps and is inefficient, which also increases sequencing time and sequencing cost.

In the third method, designs of the fluid system and the reverse loading of samples need to be further optimized.

### SUMMARY

In order to overcome or alleviate at least one or more above-mentioned technical problems existing in the prior art, there is a need to provide a fluid system, a fluid transport method, a gene sequencer and a biochemical assay method.

According to an aspect of the embodiments of the present disclosure, a fluid transport method is provided, including:
step a) of aspirating a first fluid from a fluid cartridge by using a pump valve assembly, so that the first fluid is input from an outlet of a flow cell into a lane of the flow cell, and the first fluid output from an inlet of the flow cell is returned to the fluid cartridge; and/or
step b) of aspirating a second fluid from a fluid cartridge by using a pump valve assembly, so that the second fluid is input from the inlet of the flow cell into the lane of the flow cell, and the second fluid output from the outlet of the flow cell is returned to the fluid cartridge.

In some exemplary embodiments, the pump valve assembly includes a fluid driving assembly configured to provide a positive pressure driving force or a negative pressure driving force in the step a) and the step b); and the step a) includes: a positive pressure driving step of generating the positive pressure driving force on an outlet side of the flow cell so that the first fluid is input into the lane from the outlet; or a negative pressure driving step of generating the negative pressure driving force on an inlet side of the flow cell so that the first fluid is input into the lane from the outlet.

In some exemplary embodiments, the pump valve assembly further includes a first fluid loading module configured to be switchable at least between a first position for fluidly connecting the fluid driving assembly to the fluid cartridge and a second position for fluidly connecting the fluid driving assembly to the outlet of the flow cell; and the step a) includes: firstly switching the first fluid loading module to the first position so that the negative pressure driving force is generated to aspirate the first fluid from the fluid cartridge, and then switching the first fluid loading module to the second position so that the positive pressure driving step is performed on the outlet side of the flow cell.

In some exemplary embodiments, the pump valve assembly further includes a second fluid loading module configured to be switchable at least between a first position for fluidly connecting the inlet of the flow cell to the fluid cartridge and a second position for fluidly connecting the inlet of the flow cell to the fluid driving assembly; the first fluid loading module is further configured to be switchable between the first position, the second position, and a third position for fluidly connecting the fluid cartridge to the outlet of the flow cell; and the step a) includes: firstly switching the first fluid loading module to the first position so that the negative pressure driving force is generated to aspirate the first fluid from the fluid cartridge, and then switching the first fluid loading module to the second position and switching the second fluid loading module to the first position so that the positive pressure driving step is performed on the outlet side of the flow cell; or switching the first fluid loading module to the third position and switching the second fluid loading module to the second position so that the negative pressure driving step is performed on the inlet side of the flow cell.

In some exemplary embodiments, the fluid cartridge includes a sample cartridge and a first reagent kit arranged in parallel, and the fluid transport method further includes: selectively performing a fluid transport of a sample in the sample cartridge or a fluid transport of a reagent in the first reagent kit.

In some exemplary embodiments, a second reagent kit in fluid communication with the second fluid loading module is provided, and the second fluid loading module is further configured to be switchable between the first position, the second position, and a third position for fluidly connecting the second reagent kit to the inlet of the flow cell; and the step b) includes: switching the first fluid loading module to the second position and switching the second fluid loading module to the third position, so that the negative pressure driving force is generated on the outlet side of the flow cell to input a reagent in the second reagent kit into the lane of the flow cell from the inlet.

In some exemplary embodiments, a fluid drive control assembly is provided, and the fluid transport method further includes: controlling at least one of the fluid driving assembly, the first fluid loading module or the second fluid loading module through the fluid drive control assembly.

In some exemplary embodiments, a sample cartridge/reagent kit recovery module is provided, and the fluid transport method further includes: recovering the sample cartridge and/or the first reagent kit through the sample cartridge/reagent kit recovery module.

In some exemplary embodiments, the fluid cartridge further includes a cleaning module, a cleaning liquid/pure water storage module in fluid communication with the cleaning module, and a waste liquid storage module in fluid communication with the cleaning module, and the fluid transport method further includes: performing cleaning of the lane of the flow cell by using the cleaning module, the cleaning liquid/pure water storage module and the waste liquid storage module.

In some exemplary embodiments, the cleaning module is arranged in parallel with the sample cartridge and the first reagent kit, and the fluid transport method further includes: selectively performing the fluid transport of the sample in the sample cartridge, the fluid transport of the reagent in the first reagent kit, or the cleaning of the lane of the flow cell; and performing the cleaning of the lane of the flow cell through the positive pressure driving step or the negative pressure driving step in the step a) or through the step b) when the cleaning of the lane of the flow cell is selectively performed.

In some exemplary embodiments, a first mechanical motion platform, a second mechanical motion platform and a mechanical motion control assembly are provided, at least the flow cell is placed on the first mechanical motion platform, and at least the sample cartridge, the first reagent kit and the cleaning module are placed on the second mechanical motion platform; and the fluid transport method further includes: controlling a multi-axis motion of the first mechanical motion platform through the mechanical motion control assembly to connect the flow cell to a fluid transport pipeline network; and controlling a multi-axis motion of the second mechanical motion platform through the mechanical motion control assembly to selectively connect one of the sample cartridge, the first reagent kit or the cleaning module to the fluid transport pipeline network.

In some exemplary embodiments, the first fluid is different from the second fluid, the first fluid is selected from a sample, a reagent, a cleaning liquid or pure water, and the second fluid is selected from a reagent, a cleaning liquid or pure water.

According to another aspect of the embodiments of the present disclosure, a fluid system is provided, including:
a flow cell, including a plurality of lanes, inlets and outlets;
a sample cartridge and a first reagent kit, where the sample cartridge is configured to store a sample, and the first reagent kit is configured to store a reagent for sequencing;
a fluid driving assembly, configured to generate a driving force to transport the sample or the reagent in the fluid system; and
a first fluid loading module on an outlet side of the flow cell, where the first fluid loading module is configured to be switchable at least between a first position for fluidly connecting the fluid driving assembly to the fluid cartridge, a second position for fluidly connecting the fluid driving assembly to the outlet of the flow cell, and a third position for fluidly connecting the fluid cartridge to the outlet of the flow cell.

In some exemplary embodiments, the first fluid loading module includes: a first valve group consisting of a plurality of first valves, a second valve group consisting of a plurality of second valves, and a first pipette assembly consisting of a plurality of first pipettes, where the plurality of first valves, the plurality of second valves and the plurality of first pipettes correspond to the plurality of lanes of the flow cell respectively; where each first valve is connected to the fluid driving assembly, a corresponding first pipette and a corresponding lane of the flow cell, and each first valve is configured to be switchable at least between a first position for fluidly connecting the fluid driving assembly to the first pipette and a second position for fluidly connecting the fluid driving assembly to the outlet of the corresponding lane of the flow cell; and where each second valve is connected to the fluid driving assembly, a corresponding first valve and a corresponding lane of the flow cell, and each second valve is configured to be switchable at least between a first position for connecting the fluid driving assembly to the corresponding first valve and a second position for directly fluidly connecting the fluid driving assembly to the outlet of the corresponding lane of the flow cell.

In some exemplary embodiments, the first fluid loading module further includes: a plurality of groups of sensing assemblies, where each group of sensing assemblies is connected between the fluid driving assembly and an outlet of a corresponding lane of the flow cell, and each group of sensing assemblies is configured to monitor fluid characteristics.

In some exemplary embodiments, the fluid system further includes: a second fluid loading module on the inlet side of the flow cell, where the second fluid loading module is configured to be switchable at least between a first position for fluidly connecting the inlet of the flow cell to the fluid cartridge and a second position for fluidly connecting the inlet of the flow cell to the fluid driving assembly.

In some exemplary embodiments, the fluid system further includes: at least one second reagent kit on the inlet side of the plurality of lanes of the flow cell, where the second fluid loading module is arranged between the inlets of the plurality of lanes of the flow cell and the at least one second reagent kit.

In some exemplary embodiments, the second fluid loading module further includes: a solenoid valve, a third valve group consisting of a plurality of third valves, a second pipette assembly consisting of a plurality of second pipettes, and a manifold assembly, where the plurality of third valves and the plurality of second pipettes correspond to the plurality of lanes of the flow cell respectively, and the plurality of second pipettes are connected to the at least one second reagent kit and are in fluid communication with the plurality of lanes of the flow cell via the manifold assembly; where each third valve has a first port connected to the manifold assembly and a second port connected to a corresponding second pipette, and each third valve is configured to be switchable between a first position for closing fluid communication between the manifold assembly and the corresponding second pipette and a second position for opening the fluid communication between the manifold assembly and the corresponding second pipette; and where the plurality of lanes of the flow cell are connected to the sample cartridge or the first reagent kit via the solenoid valve.

In some exemplary embodiments, the fluid system further includes: a fluid drive control assembly configured to control at least one of the fluid driving assembly, the first fluid loading module or the second fluid loading module.

In some exemplary embodiments, the fluid system further includes: a sample cartridge/reagent kit recovery module configured to recover the sample cartridge and/or the first reagent kit.

In some exemplary embodiments, the fluid system further includes: a cleaning module between the fluid driving assembly and the first fluid loading module, where the cleaning module is configured to perform cleaning of the plurality of lanes of the flow cell; a cleaning liquid/pure water storage module in fluid communication with the cleaning module and the second fluid loading module, where the cleaning liquid/pure water storage module is configured to provide cleaning liquid and/or pure water when the cleaning of the plurality of lanes of the flow cell is performed; and a waste liquid storage module in fluid communication with the cleaning module and the fluid driving assembly, where the waste liquid storage module is configured to recover and/or discharge waste liquid.

In some exemplary embodiments, the fluid driving assembly includes: a syringe having a plurality of independent channels, and a plurality of reversing valves; where the plurality of independent channels and the plurality of reversing valves correspond to the plurality of lanes of the flow cell respectively; and where the plurality of reversing valves are controllable individually or simultaneously.

In some exemplary embodiments, the fluid system further includes: a first mechanical motion platform, a second mechanical motion platform, and a mechanical motion control assembly, where at least the flow cell is placed on the first mechanical motion platform, and at least the sample cartridge, the first reagent kit and the cleaning module are placed on the second mechanical motion platform; where the mechanical motion control assembly is configured to control a multi-axis motion of the first mechanical motion platform to selectively connect the flow cell to a fluid transport pipeline network of the fluid system; and where the mechanical motion control assembly is further configured to control a multi-axis motion of the second mechanical motion platform to selectively connect one of the sample cartridge, the first reagent kit or the cleaning module to the fluid transport pipeline network of the fluid system.

In some exemplary embodiments, at least one of the first mechanical motion platform or the second mechanical motion platform is a three-dimensional motion platform or a rotary motion platform.

In some exemplary embodiments, the plurality of first valves in the first valve group, the plurality of second valves in the second valve group and the plurality of third valves in the third valve group are solenoid valves.

In other exemplary embodiments, the plurality of first valves in the first valve group are multi-way rotary valves, and the plurality of second valves in the second valve group and the plurality of third valves in the third valve group are solenoid valves. For example, the multi-way rotary valve is a 2-position 8-way rotary valve or a 2-position 12-way rotary valve.

In still other exemplary embodiments, the fluid system further includes: a first rotary valve on the outlet side of the plurality of lanes of the flow cell, where the first rotary value is configured to selectively connect one of the sample cartridge or the first reagent kit to the fluid transport pipeline network of the fluid system; and a second rotary valve on the inlet side of the plurality of lanes of the flow cell, where the second rotary valve is configured to selectively connect one of the sample cartridge, the first reagent kit or the second reagent kit to the fluid transport pipeline network of the fluid system.

In some optional embodiments, the fluid system further includes: a sequencing platform between the first fluid loading module and the second fluid loading module; a loading platform between the sample cartridge and the fluid driving assembly and between the first reagent kit and the fluid driving assembly; and an automatic transfer device configured to automatically transfer the flow cell between the sequencing platform and the loading platform.

According to another aspect of the embodiments of the present disclosure, a gene sequencer is provided, including:
a flow cell, including a plurality of lanes, inlets and outlets;
a sample cartridge and a first reagent kit, where the sample cartridge is configured to store a sample, and the first reagent kit is configured to store a reagent for sequencing;
a fluid driving assembly configured to generate a driving force to transport the sample or the reagent in the fluid system; and
a first fluid loading module on an outlet side of the flow cell, where the first fluid loading module is configured to be switchable at least between a first position for fluidly connecting the fluid driving assembly to the sample cartridge or the first reagent kit, a second position for fluidly connecting the fluid driving assembly to the outlet of the flow cell, and a third position for fluidly connecting the sample cartridge or the first reagent kit to the outlet of the flow cell.

In some exemplary embodiments, the first fluid loading module includes: a first valve group consisting of a plurality of first valves, a second valve group consisting of a plurality of second valves, and a first pipette assembly consisting of a plurality of first pipettes, where the plurality of first valves, the plurality of second valves and the plurality of first pipettes correspond to the plurality of lanes of the flow cell respectively; where each first valve is connected to the fluid driving assembly, a corresponding first pipette and a corresponding lane of the flow cell, and each first valve is configured to be switchable at least between a first position for fluidly connecting the fluid driving assembly to the first pipette and a second position for fluidly connecting the fluid driving assembly to the outlet of the corresponding lane of the flow cell; and where each second valve is connected to the fluid driving assembly, a corresponding first valve and a corresponding lane of the flow cell, and each second valve is configured to be switchable at least between a first position for connecting the fluid driving assembly to the corresponding first valve and a second position for directly fluidly connecting the fluid driving assembly to the outlet of the corresponding lane of the flow cell.

In some exemplary embodiments, the first fluid loading module further includes: a plurality of groups of sensing assemblies, where each group of sensing assemblies is connected between the fluid driving assembly and an outlet of a corresponding lane of the flow cell, and each group of sensing assemblies is configured to monitor fluid characteristics.

In some exemplary embodiments, the gene sequencer further includes: a second fluid loading module on the inlet side of the flow cell, where the second fluid loading module is configured to be switchable at least between a first position for fluidly connecting the inlet of the flow cell to the sample cartridge or the first reagent kit and a second position for fluidly connecting the inlet of the flow cell to the fluid driving assembly.

In some exemplary embodiments, the gene sequencer further includes: at least one second reagent kit on the inlet side of the plurality of lanes of the flow cell, where the second fluid loading module is arranged between the inlets of the plurality of lanes of the flow cell and the at least one second reagent kit.

In some exemplary embodiments, the second fluid loading module further includes: a solenoid valve, a third valve group consisting of a plurality of third valves, a second pipette assembly consisting of a plurality of second pipettes, and a manifold assembly, where the plurality of third valves and the plurality of second pipettes correspond to the plurality of lanes of the flow cell respectively, and the plurality of second pipettes are connected to the at least one second reagent kit and are in fluid communication with the plurality of lanes of the flow cell via the manifold assembly; and where each third valve has a first port connected to the manifold assembly and a second port connected to a corresponding second pipette, and each third valve is configured to be switchable between a first position for closing fluid communication between the manifold assembly and the corresponding second pipette and a second position for opening the fluid communication between the manifold assembly and the corresponding second pipette; and where the plurality of lanes of the flow cell are connected to the sample cartridge or the first reagent kit via the solenoid valve.

In some exemplary embodiments, the gene sequencer further includes: a fluid drive control assembly configured to control at least one of the fluid driving assembly, the first fluid loading module or the second fluid loading module.

In some exemplary embodiments, the gene sequencer further includes: a sample cartridge/reagent kit recovery module configured to recover the sample cartridge and/or the first reagent kit.

In some exemplary embodiments, the gene sequencer further includes: a cleaning module between the fluid driving assembly and the first fluid loading module, where the cleaning module is configured to perform cleaning of the plurality of lanes of the flow cell; a cleaning liquid/pure water storage module in fluid communication with the cleaning module and the second fluid loading module, where the cleaning liquid/pure water storage module is configured to provide cleaning liquid and/or pure water when the cleaning of the plurality of lanes of the flow cell is performed; and a waste liquid storage module in fluid communication with the cleaning module and the fluid driving assembly, where the waste liquid storage module is configured to recover and/or discharge waste liquid.

In some exemplary embodiments, the fluid driving assembly includes: a syringe having a plurality of independent channels, and a plurality of reversing valves; where the plurality of independent channels and the plurality of reversing valves correspond to the plurality of lanes of the flow cell respectively; and where the plurality of reversing valves are controllable individually or simultaneously.

In some exemplary embodiments, the gene sequencer further includes: a first mechanical motion platform, a second mechanical motion platform, and a mechanical motion control assembly, where at least the flow cell is placed on the first mechanical motion platform, and at least the sample cartridge, the first reagent kit and the cleaning module are placed on the second mechanical motion platform; where the mechanical motion control assembly is configured to control a multi-axis motion of the first mechanical motion platform to selectively connect the flow cell to the fluid transport pipeline network of the fluid system; and where the mechanical motion control assembly is further configured to control a multi-axis motion of the second mechanical motion platform to selectively connect one of the sample cartridge, the first reagent kit or the cleaning module to the fluid transport pipeline network of the fluid system.

In some exemplary embodiments, at least one of the first mechanical motion platform or the second mechanical motion platform is a three-dimensional motion platform or a rotary motion platform.

In some exemplary embodiments, the plurality of first valves in the first valve group, the plurality of second valves in the second valve group and the plurality of third valves in the third valve group are solenoid valves.

In other exemplary embodiments, the plurality of first valves in the first valve group are multi-way rotary valves, and the plurality of second valves in the second valve group and the plurality of third valves in the third valve group are solenoid valves. For example, the multi-way rotary valve is a 2-position 8-way rotary valve or a 2-position 12-way rotary valve.

In still other exemplary embodiments, the gene sequencer further includes: a first rotary valve on the outlet side of the plurality of lanes of the flow cell, where the first rotary value is configured to selectively connect one of the sample cartridge or the first reagent kit to the fluid transport pipeline network of the fluid system; and a second rotary valve on the inlet side of the plurality of lanes of the flow cell, where the second rotary value is configured to selectively connect one of the sample cartridge, the first reagent kit or the second reagent kit to the fluid transport pipeline network of the fluid system.

In some optional embodiments, the gene sequencer further includes: a sequencing platform between the first fluid loading module and the second fluid loading module; a loading platform between the sample cartridge and the fluid driving assembly and between the first reagent kit and the fluid driving assembly; and an automatic transfer device configured to automatically transfer the flow cell between the sequencing platform and the loading platform.

According to still another aspect of the present disclosure, a biochemical assay method is provided, including: loading a sample to be detected into a lane of a flow cell from an outlet side of the flow cell; and loading a reagent containing a plurality of different reaction components into the lane of the flow cell from an inlet side or the outlet side of the flow cell, so as to perform a biochemical reaction between the sample and the reagent, where the reaction component includes at least one of a sample generation component or a sample analysis component; where the biochemical reaction includes: generating a sample in the lane of the flow cell, by allowing different sample generation components to flow into the lane and controlling a reaction condition in the lane; and the biochemical reaction further includes: analyzing the sample in the lane, by allowing the sample analysis component to flow into the lane and react with the sample to provide a related detectable signal.

In some exemplary embodiments, in the biochemical assay method, the biochemical reaction is a nucleic acid sequencing reaction, and the sample to be detected is a nucleic acid sequencing library.

In some exemplary embodiments, in the biochemical assay method, the detectable signal is an optical signal.

In some exemplary embodiments, the biochemical assay method further includes: in a case of loading the reagent into the lane of the flow cell from a first reagent kit on the outlet side of the flow cell, discharging the reagent to the first reagent kit via the inlet of the flow cell after performing the biochemical reaction; or in a case of loading the reagent into the lane of the flow cell from a second reagent kit on the inlet side of the flow cell, pushing the reagent back to the second reagent kit via the inlet of the flow cell after performing the biochemical reaction.

In some exemplary embodiments, the biochemical assay method further includes: loading cleaning liquid/pure water into the lane of the flow cell from the outlet side of the flow cell, and discharging the cleaning liquid/pure water to the first reagent kit, the second reagent kit or a waste liquid storage module via the inlet of the flow cell; or loading the cleaning liquid/pure water into the lane of the flow cell from the inlet side of the flow cell, and pushing the cleaning liquid/pure water back to the second reagent kit or the waste liquid storage module via the inlet of the flow cell.

According to yet another aspect of the present disclosure, a mechanical motion device for a fluid system is provided, where the fluid system includes at least a sample cartridge, a reagent kit and a flow cell; the mechanical motion device including:
a first mechanical motion platform, where the flow cell is placed on the first mechanical motion platform;
a second mechanical motion platform, where the sample cartridge and the reagent kit are placed on the second mechanical motion platform; and
a mechanical motion control assembly configured to: control a multi-axis motion of the first mechanical motion platform to connect the flow cell to a fluid transport pipeline network of the fluid system, and control a multi-axis motion of the second mechanical motion platform to selectively connect one of the sample cartridge or the reagent kit to the fluid transport pipeline network.

In some exemplary embodiments, the fluid system further includes a cleaning module, and the cleaning module is placed on the second mechanical motion platform; and the mechanical motion control assembly is further configured to control the multi-axis motion of the second mechanical motion platform to selectively connect one of the sample cartridge, the reagent kit or the cleaning module to the fluid transport pipeline network.

In some exemplary embodiments, at least one of the first mechanical motion platform or the second mechanical motion platform is a three-dimensional motion platform or a rotary motion platform.

According to the fluid system, the fluid transport method, the gene sequencer and the biochemical assay method provided in various aspects and/or embodiments of the present disclosure, a sample or a reagent is transported in reverse from the outlet to the inlet of the flow cell, and the reversely loaded sample or reagent may eventually be directly returned to the sample cartridge or the reagent kit. Furthermore, when cleaning of the lanes of the flow cell and the fluid transport pipeline network is performed, the waste liquid after cleaning may flow directly into the reagent kit. It is also possible to load the same or different samples into different lanes of the flow cell, so that the sequencing time is reduced, and the flexibility of sample/reagent loading is improved. In addition, the sample loading system and the sequencer are integrated together by means of the control of the mechanical motion platform, then it is possible to load the same or different samples into different lanes of the sequencing chip on the gene sequencer, and it is also possible to load the reagent, so that sequencing steps are reduced, and the sequencing efficiency is improved.

With following descriptions of the present disclosure with reference to the accompanying drawings, other objectives and advantages of the present disclosure will be obvious and the present disclosure may be understood comprehensively.

### BRIEF DESCRIPTION OF THE DRAWINGS

FIG. 1 shows a schematic diagram of a fluid system provided according to an embodiment of the present disclosure;
FIG. 2 shows a schematic diagram of a fluid system according to a first exemplary embodiment of the present disclosure;
FIG. 3 shows a schematic diagram of a fluid system according to a second exemplary embodiment of the present disclosure;
FIG. 4A and FIG. 4B show partial schematic diagrams of a fluid system according to a third exemplary embodiment of the present disclosure, in which states of a multi-way rotary valve in a first position and a second position are shown respectively;
FIG. 5 shows a schematic diagram of a fluid system according to a first optional embodiment of the present disclosure;
FIG. 6A and FIG. 6B show partial schematic diagrams of the fluid system according to the first optional embodiment of the present disclosure; and
FIG. 7 shows a schematic diagram of a fluid system according to a second optional embodiment of the present disclosure.

### DETAILED DESCRIPTION OF EMBODIMENTS

Technical solutions of the present disclosure will be further described in detail below through the embodiments with reference to the accompanying drawings. In the specification, the same or similar reference numerals represent the same or similar components. The following descriptions of the embodiments of the present disclosure with reference to the accompanying drawings are intended to explain a general inventive concept of the present disclosure, and should not be understood as limitations to the present disclosure.

In addition, in the following detailed descriptions, for the convenience of explanation, many specific details are set forth to provide comprehensive understanding of the embodiments of the present disclosure. Obviously, however, one or more embodiments may also be implemented without these specific details. In other cases, well-known structures and devices are illustrated to simplify the accompanying drawings.

The embodiments of the present disclosure mainly relate to a fluid system, a fluid transport method, a gene sequencer and a biochemical assay method.

### [Fluid System]

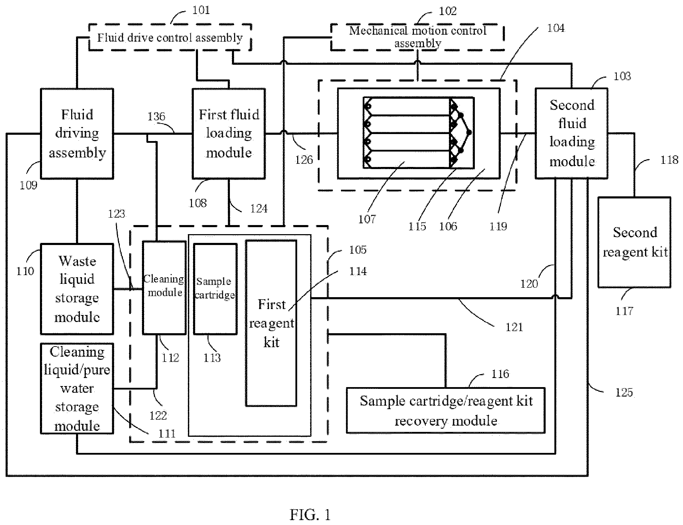

FIG. 1 shows main functional modules of a fluid system provided according to an embodiment of the present disclosure and their coordination relationships. These functional modules and their functions and/or component parts will be described below with reference to FIG. 1.

A fluid drive control assembly 101 is used to drive fluid devices. Specifically, the fluid drive control assembly 101 may separately or simultaneously drive a plurality of fluid devices such as pumps, valves, sensors, etc. in a fluid driving assembly 109, a first fluid loading module 108 and a second fluid loading module 103 to be described in detail below.

A mechanical motion control assembly 102 is used to drive a first mechanical motion platform 104 and/or a second mechanical motion platform 105 to be described in detail below, to achieve multi-axis motion of the first mechanical motion platform and/or the second mechanical motion platform. A chip-carrying platform module 106 to be described in detail below is placed on the first mechanical motion platform 104, and some devices of the first fluid loading module 108 may also be placed on the first mechanical motion platform 104. A sample cartridge 113, a first reagent kit 114 and a cleaning liquid/pure water storage module 111 to be described in detail below may be partially or completely placed on the second mechanical motion platform 105.

A fluid driving assembly 109 is used to generate a driving force under the drive of the fluid drive control assembly 101 to achieve a movement of a sample or a reagent in the fluid system. The driving force may be in a form of pressure, which includes negative pressure and positive pressure, depending on a desired direction of fluid transport. For example, it is possible to use a negative pressure driving method to aspirate a reagent in a second reagent kit 117 to be described in detail below into a lane 107 of a flow cell 115 to be described in detail below through the first fluid loading module 108 and the second fluid loading module 103, or it is possible to use a positive pressure driving method to push the reagent in the flow cell 115 back into the second reagent kit 117. The fluid driving assembly 109 has one or more fluid interfaces to achieve the transport of fluids in different lanes. For example, the fluid driving assembly 109 is in fluid communication with the first fluid loading module 108 and the second fluid loading module 103 to achieve transport of sample and reagent. For another example, the fluid driving assembly 109 is in fluid communication with a cleaning module 112 to be described in detail below and may provide a driving force for cleaning the entire fluid system. For another example, the fluid driving assembly 109 is in fluid communication with a waste liquid storage module 110 to be described in detail below and may achieve discharge of waste liquid. Furthermore, the fluid driving assembly 109 may be a hydraulic unit in the form of a peristaltic pump, a plunger pump, a syringe pump, a gear pump or a diaphragm pump, or an air driving unit in the form of a vacuum pump, a diaphragm pump or an air compressor. Furthermore, the fluid driving assembly 109 has a reversing valve, and the reversing valve may be a reversing device such as a solenoid valve, a rotary valve, a pneumatic reversing valve, an electro-hydraulic reversing valve, a manual reversing valve, a piezoelectric valve, a pinch valve, a rotary valve or a rotary cutting valve.

The first fluid loading module 108 and the second fluid loading module 103 are used to achieve switching between different fluid pipelines so that the sample or reagent is transported according to a set path, and the first fluid loading module 108 and the second fluid loading module 103 further have a sensing assembly to detect a motion state of the fluid. The first fluid loading module 108 has at least three interfaces, which may be connected to the fluid driving assembly 109, the flow cell 115 and the second mechanical motion platform 105 respectively. The second fluid loading module 103 has at least three interfaces, which may be connected to the second reagent kit 117, the flow cell 115 and the fluid driving assembly 109 respectively. The first fluid loading module and the second fluid loading module each may further include a pipette assembly, a reversing assembly, a manifold assembly and a sensing assembly. Furthermore, the pipette assembly has one or more interfaces. Each pipette may be in fluid communication with a corresponding sample cartridge, a corresponding reagent kit or the cleaning module, or may be in fluid communication with the corresponding sample cartridge, the corresponding reagent kit or the cleaning module in sequence through transport by a mechanical motion platform. An interface opening connected to the sample cartridge or the reagent kit may be vertically upward or vertically downward. Furthermore, the reversing assembly has one or more fluid direction switching functions, which may achieve opening, closing or reversing of a flow path. A device that achieves the reversing function may be a reversing device such as a solenoid valve, a rotary valve, a pneumatic reversing valve, an electro-hydraulic reversing valve, a manual reversing valve, a piezoelectric valve, a pinch valve, a rotary valve or a rotary cutting valve. Furthermore, the manifold assembly is a pipeline that may collect a plurality of pipelines together to achieve collection or diversion of samples/reagents. Furthermore, the sensing assembly may be a pressure sensor, a flow sensor, a bubble sensor, or the like.

The chip-carrying platform module 106 is used to carry the flow cell 115 and interfaces 141 and 142 connected to the outside, and may drive the flow cell 115 to perform a multi-axis motion.

The flow cell 115 is a place where a biochemical reaction is performed during sequencing, and has one or more lanes 107. Each lane may be exactly the same or different in size. Furthermore, the plurality of lanes may converge into one lane at the outlet or the inlet.

The waste liquid storage module 110 has one or more interfaces and may be used to store or discharge all or part of waste liquid of a liquid system. The waste liquid storage module may be located inside or outside the instrument, or the waste liquid may be directly discharged to a laboratory waste liquid treatment system through a waste liquid interface. Furthermore, the waste liquid storage module 110 may include no container, or may include one or more containers. Furthermore, according to functional requirements, the waste liquid storage module 110 may include a sensor for liquid detection, an air purification device or a secondary overflow prevention device, etc.

A cleaning liquid/pure water storage module 111 is used to store pure water or cleaning liquid required for cleaning the entire system. The cleaning liquid and pure water may be packaged separately or integrated together, and may be fixed on the second mechanical motion platform 105 or may be taken and placed independently. Furthermore, according to functional requirements, the cleaning liquid/pure water storage module 111 may include a liquid volume detection sensor, an air purification device, etc. Furthermore, the cleaning liquid/pure water storage module 111 may also be an interface directly connected to the outside of the instrument.

The sample cartridge 113 is used to store samples and may be in fluid communication with the first fluid loading module 108. The sample cartridge 113 has one or more hole positions, which correspond to one or more lanes 107 of the flow cell 115 respectively. Furthermore, the sample cartridge 113 may receive recovered samples or reagents, and it is also possible to add one or more empty hole positions for recovery.

The first reagent kit 114 and the second reagent kit 117 are used to store reagents required by the system during sequencing. The first reagent kit 114 may include one or more reagent kits located on the second mechanical motion platform 105, and the reagents may be loaded into the flow cell 115 through the first fluid loading module 108. The second reagent kit 117 may include one or more reagent kits, and the reagents may be loaded into the flow cell 115 or the waste liquid storage module 110 through the second fluid loading module 103.

A sample cartridge/reagent kit recovery module 116 is used to store and recover the sample cartridge and the reagent kit after use.

### [Fluid Transport Method]

For ease of description, in FIG. 1 and the figures to be mentioned below, a flow of fluid from right to left in the flow cell 115 is defined as a forward flow, and a flow from left to right is defined as a reverse flow.

The fluid transport methods according to the embodiments of the present disclosure may be generally summarized as follows.

### 1. Reverse loading and transport of fluid

The fluid may be a sample, a reagent, a cleaning liquid, or pure water. The sample/reagent may enter the flow cell 115 to perform a biochemical reaction, and the cleaning liquid/pure water may enter the flow cell 115 and its upstream and downstream to clean the fluid system.

The reverse loading and transport of fluid may be implemented by two methods. For ease of understanding, reverse loading and transport of sample is used as an example for description below.

A first method is a positive pressure driving method. Under the control of the first mechanical motion platform 104 or the second mechanical motion platform 105, the first fluid loading module 108 is switched to be in fluid communication with the sample cartridge 113 and the fluid driving assembly 109, and the fluid driving assembly 109 provides negative pressure to aspirate the sample into the pipeline 136 for buffering. After the liquid aspiration is completed, the first fluid loading module 108 is switched to be in fluid communication with the fluid driving assembly 109 and the flow cell 115. The fluid driving assembly 109 is switched to provide positive pressure to push the sample buffered in the pipeline 136 into one or more lanes 107 of the flow cell 115 through the first fluid loading module 108. Excess fluid may be discharged into the sample cartridge 113 or into the waste liquid storage module 110 as required.

A second method is a negative pressure driving method. Under the control of the first mechanical motion platform 104 or the second mechanical motion platform 105, the first fluid loading module 108 is switched to be in fluid communication with the sample cartridge 113 and the flow cell 115, and the second fluid loading module 103 is switched to be in fluid communication with the fluid driving assembly 109 and the flow cell 115. The fluid driving assembly 109 provides negative pressure to aspirate the sample directly into one or more lanes 107 of the flow cell 115. Excess fluid may be discharged into the sample cartridge 113 or into the waste liquid storage module 110 as required.

The above-mentioned two reverse loading and transport methods may be performed to load different samples or the same sample into the lanes 107 of the flow cell.

### 2. Forward loading and transport of fluid

For ease of understanding, forward loading and transport of reagent is used as an example for description below.

The first fluid loading module 108 is switched to be in fluid communication with the fluid driving assembly 109 and the flow cell 115, and the second fluid loading module 103 is switched to be in fluid communication with the second reagent kit 117 and the flow cell 115. The fluid driving assembly 109 provides a driving force to aspirate a reagent in the second reagent kit 117 into the flow cell 115, and excess reagent is discharged into the waste liquid storage module 110. Such method may also be performed to load the same sample or clean the entire liquid system.

In addition, when the second fluid loading module 103 is switched to be in fluid communication with the fluid driving assembly 109 and the second reagent kit 117, it is also possible to perform fluid perfusion and cleaning of the second fluid loading module 103.

### [First Exemplary Embodiment]

### Fluid System

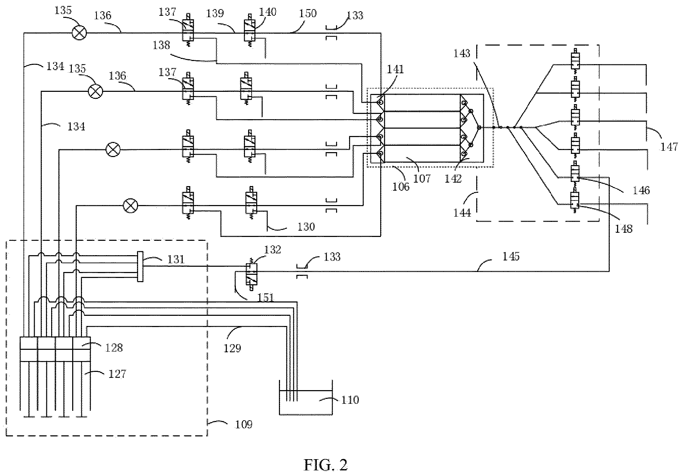

FIG. 2 shows a schematic diagram of a fluid system according to a first exemplary embodiment of the present disclosure.

As shown in FIG. 2, in this embodiment, the fluid driving assembly 109 is an injection pump assembly including a syringe 127 and a reversing valve 128. The injection pump assembly has a plurality of independent channels, and the reversing valve 128 has a plurality of interfaces that may be switched to different pipelines. Each channel may be controlled individually or simultaneously.

Referring to FIG. 2 in combination with FIG. 1, the first fluid loading module 108 includes a first valve group 140, a second valve group 137, a pipette assembly 130, a bubble sensing assembly 133, and a pressure sensing assembly 135. The first fluid loading module 108 corresponds to the lanes 107 of the flow cell 115 on the chip-carrying platform module 106 respectively. For example, a solenoid valve in the first valve group 140, a solenoid valve in the second valve group 137 and a corresponding pipette in the pipette assembly 130, etc. all correspond to a lane 107. Each solenoid valve in the first valve group 140 has at least three interfaces, which are in fluid communication with the corresponding pipette assembly 130, a pipeline 139 and a pipeline 150 respectively. Each solenoid valve in the second valve group 137 has at least three interfaces, which are in fluid communication with the pipeline 139, a pipeline 136 and a pipeline 138 respectively. The bubble sensor 133 and the pressure sensor 135 are located between the syringe 127 and the lane 107 of the flow cell. Here, the pressure sensing assembly 135 may be a flow sensor assembly or other sensing modules capable of monitoring fluid characteristics.

The second fluid loading module 103 includes a third group of solenoid valves 148, a solenoid valve 146, a manifold assembly 144 and a pipette assembly 147. The solenoid valve 148 and the solenoid valve 146 each have two ports and are connected in parallel. The first port of the solenoid valve 148 and the first port of the solenoid valve 146 converge into a common pipeline 143. The second port of the solenoid valve 148 is in fluid communication with the pipette assembly 147, and the second port of the solenoid valve 146 is in fluid communication with a reagent pipeline 145. Here, the first port of the third valve group 148 and the common pipeline 143 may be converged in a stepped manner or in a centralized manner.

In this embodiment, a sample connection interface is located between the syringe pump and the outlet of the flow cell. Two or more different reagents may be located between the syringe pump 127 and the outlet of the flow cell 105 and at the inlet of the flow cell 105 respectively. In this embodiment, as shown in FIG. 2, the illustrated positions of solenoid valves 140, 137, 148 and 146 are the first positions by default, which may be switched to the second positions as required.

### Fluid Transport Method

### First fluid transport method: transport of sample/reagent (reverse transport)

This fluid transport method may be implemented to transport samples or reagents. When transporting samples, the fluid transport method may be implemented to load different samples into different lanes of the flow cell, and may also be implemented to load the same sample into different lanes of the flow cell.

Referring to FIG. 2 in combination with FIG. 1, the process and steps of the fluid transport method will be described below.

In step 1), the second mechanical motion platform 105 carries the sample cartridge 113 to a position corresponding to the pipette assembly 130 in the first fluid loading module 108, a solenoid valve in the first valve group 140 is switched to the second position, and a corresponding pipette in the pipette assembly 130 is in fluid communication with a first sample in the sample cartridge 113.

In step 2), the reversing valve 128 in the fluid driving assembly 109 is switched to be in fluid communication with the pipeline 134, and the syringe 127 aspirates the first sample in the sample cartridge 113 to the pipeline 136 for buffering through a solenoid valve in the first valve group 140 and a corresponding pipette in the pipette assembly 130.

In step 3), the solenoid valve 146 in the second fluid loading module 103 is switched to the second position, a solenoid valve in the first valve group 140 is switched to the first position, and the pipette assembly 151 is in fluid communication with the sample cartridge 113. The syringe 127 pushes the sample in the pipeline 136 to a corresponding lane 107 of the flow cell 115 along a solenoid valve in the first valve group 140, the fluid pipeline 150 and a flow cell interface 141. The sample/reagent in the pipeline 145 may be recovered into the sample cartridge 113. On the other hand, if the pipette assembly 151 is in fluid communication with the waste liquid storage module 110, the sample/reagent in the pipeline 145 may be discharged to the waste liquid storage module.

Continuing to refer to FIG. 2 in combination with FIG. 1, the transport of reagent from the first reagent kit 114 will be described below, and an operation flow is substantially the same as the above-mentioned operation flow of the sample.

In step 4), the second mechanical motion platform 105 carries the first reagent kit 114 to a position corresponding to the pipette assembly 130 in the first fluid loading module 108, a solenoid valve in the first valve group 140 is switched to the second position, and a corresponding pipette in the pipette assembly 130 is in fluid communication with a first reagent in the reagent kit 114.

In step 5), the reversing valve 128 in the fluid driving assembly 109 is switched to be in fluid communication with the pipeline 134, and the syringe 127 aspirates the reagent in the first reagent kit 114 to the pipeline 136 for buffering through a solenoid valve in the first valve group 140 and a corresponding pipette in the pipette assembly 130.

In step 6), the solenoid valve 146 in the second fluid loading module 103 is switched to the second position, the first valve group 140 is switched to the first position, and the pipette assembly 151 is in fluid communication with the first reagent kit 114. The syringe 127 pushes the reagent in the pipeline 136 to a corresponding lane 107 of the flow cell 115 along a solenoid valve in the first valve group 140, the fluid pipeline 150 and the flow cell interface 141. Excess reagent in the pipeline 145 may be recovered into the first reagent kit 114. On the other hand, if the pipette assembly 151 is in fluid communication with the waste liquid storage module 110, the sample/reagent in the fluid pipeline 145 may be discharged to the waste liquid storage module 110.

Second fluid transport method: transport of reagent (reagent used for sequencing is input from a front end of the flow cell).

Continuing to refer to FIG. 2 in combination with FIG. 1, a process and steps of the fluid transport method will be described below.

In step 1), i.e., a reagent perfusion process, a corresponding pipette in the pipette assembly 147 is in fluid communication with a corresponding first reagent in a second reagent kit 117. The solenoid valve 146, the solenoid valve 132 and a solenoid valve in the third valve group 148 are switched to the second position, the reversing valve 128 in the fluid driving assembly 109 is switched to be in fluid communication with the manifold assembly 131, and the syringe 127 provides negative pressure to aspirate the first reagent in the second reagent kit 117 into the manifold assembly 144 and the fluid pipeline 145. The reversing valve 128 in the fluid driving assembly 109 is switched to be in fluid communication with the fluid pipeline 129, and the syringe 127 provides positive pressure to discharge the reagent into the waste liquid storage module 110.

In step 2), i.e., a sequencing process, a corresponding pipette in the pipette assembly 147 is in fluid communication with the first reagent in the second reagent kit 117, a solenoid valve in the third valve group 148 is switched to the second position, and the reversing valve 128 in the fluid driving assembly 109 is switched to be in fluid communication with the pipeline 134. The syringe 127 aspirates the first reagent and transports it along a corresponding pipette in the pipette assembly 147, a solenoid valve in the third valve group 148, the common pipeline 143, the lane 107 in the flow cell 115, and the fluid pipeline 150. After the first reagent completes a biochemical reaction in the lane 107 of the flow cell 115, the reversing valve 128 is switched to be in fluid communication with the pipeline 129, and the syringe 127 discharges the reacted reagent into the waste liquid storage module 110.

Another corresponding pipette in the pipette assembly 147 is in fluid communication with the second reagent in the second reagent kit 117, another solenoid valve in the third valve group 148 is switched to the second position, and the reversing valve 128 in the fluid driving assembly 109 is switched to be in fluid communication with the pipeline 134. The second valve group 137 is switched to the second position, and the syringe 127 aspirates the second reagent and transports it along another corresponding pipette in the pipette assembly 147, another solenoid valve in the third valve group 148, the common pipeline 143, the lane 107 of the flow cell 115, and the pipeline 150. After the second reagent completes a biochemical reaction in the lane 107 of the flow cell 115, the reversing valve 128 is switched to be in fluid communication with the pipeline 129, and the syringe 127 discharges the reacted reagent into the waste liquid storage module 110.

In step 3), i.e., a sequencing reagent recovery process, a corresponding pipette in the pipette assembly 147 is in fluid communication with the first reagent in the second reagent kit 117, a solenoid valve in the third valve group 148 is switched to the second position, and the reversing valve 128 in the fluid driving assembly 109 is switched to be in fluid communication with the pipeline 134. The syringe 127 aspirates the first reagent and transports it along a corresponding pipette in the pipette assembly 147, a solenoid valve in the third valve group 148, the common pipeline 143, the lane 107 of the flow cell 115, and the pipeline 150. After the first reagent completes a biochemical reaction in the lane 107 of the flow cell 115, the syringe 127 directly pushes the first reagent back to a corresponding hole position of the second reagent kit 117.

Another corresponding pipette in the pipette assembly 147 is in fluid communication with the second reagent in the second reagent kit 117, another solenoid valve in the third valve group 148 is switched to the second position, and the reversing valve 128 in the fluid driving assembly 109 is switched to be in fluid communication with the pipeline 134. The second valve group 137 is switched to the second position, and the syringe 127 aspirates the second reagent and transports it along another corresponding pipette in the pipette assembly 147, another solenoid valve in the third valve group 148, the common pipeline 143, the lane 107 in the flow cell 115, and the pipeline 150. After the second reagent completes the biochemical reaction in the lane 107 of the flow cell 115, the syringe 127 directly pushes the second reagent back to a corresponding hole position of the second reagent kit 117.

### Third fluid transport method: pipeline cleaning.

Continuing to refer to FIG. 2 in combination with FIG. 1, a path and method for cleaning the pipeline will be described below.

In a first cleaning path, cleaning liquid/pure water is aspirated from the pipette assembly 130, and discharged to the waste liquid storage module 110 via the pipeline 129 or discharged to the second reagent kit 117 via the pipeline 147.

Specifically, the second mechanical motion platform 105 carries the cleaning module 112 to a position corresponding to the pipette assembly 130, the first valve group 140 is switched to the second position, the reversing valve 128 is switched to be in fluid communication with the pipeline 134, and the syringe 127 aspirates the cleaning liquid/pure water from the cleaning module 112 into the syringe 127 along the pipette assembly 130, the first valve group 140, the second valve group 137, the pipeline 136 and the pipeline 134.

A) In a first discharge and cleaning method, after the liquid aspiration is completed, the reversing valve 128 is switched to be in fluid communication with the pipeline 129, and the syringe 127 discharges the waste liquid into the waste liquid storage module 110.

B) In a second discharge and cleaning method, after the liquid aspiration is completed, the first valve group 140 is switched to the first position, the third valve group 148 is switched to the second position, and the syringe 127 discharges the waste liquid into the second reagent kit 117 along the pipeline 134, the pipeline 150, the lane 107 in the flow cell 115, and the pipette assembly 147.

C) In a third discharge and cleaning method, after the liquid aspiration is completed, the second valve group 137 is switched to the second position, the third valve group 148 is switched to the second position, and the syringe 127 discharges the waste liquid into the second reagent kit 117 along the pipeline 134, the pipeline 138, the lane 107 in the flow cell 115, and the pipette assembly 147.

D) In a fourth discharge and cleaning method, after the liquid aspiration is completed, the first valve group 140 is switched to the first position, the solenoid valve 146 is switched to the second position, and the syringe 127 discharges the waste liquid into the first reagent kit 114 or the second reagent kit 117 along the pipeline 134, the pipeline 150, the lane 107 in the flow cell 115, and the pipette assembly 151.

E) In a fifth discharge and cleaning method, after the liquid aspiration is completed, the second valve group 137 is switched to the first position, the solenoid valve 146 is switched to the second position, and the syringe 127 discharges the waste liquid into the first reagent kit 114 or the second reagent kit 117 along the pipeline 134, the pipeline 138, the lane 107 in the flow cell 115, and the pipette assembly 151.

In a second cleaning path, the cleaning liquid/pure water is aspirated from the pipette assembly 147, and discharged from the pipeline 129 to the waste liquid storage module 110 or discharged from the pipette assembly 130 to the first reagent kit 114.

Specifically, the second mechanical motion platform 105 carries the cleaning module 112 to a position corresponding to the pipette assembly 147, and the third valve group 148 is switched to the second position. The reversing valve 128 is switched to be in fluid communication with the pipeline 134, and the syringe 127 aspirates the cleaning liquid/pure water from the cleaning module 112 to the syringe 127 for buffering via the lane 107 of the flow cell 115 and the pipeline 150. In this embodiment, if the second valve group 137 is switched to the second position, the reagent aspirated by the syringe may also be buffered in the syringe 127 via the lane 107 in the flow cell 115 and the pipeline 138.

A) In a first discharge and cleaning method, after the liquid aspiration is completed, the reversing valve 128 is switched to be in fluid communication with the pipeline 129, and the syringe 127 discharges the waste liquid into the waste liquid storage module 110.

B) In a second discharge and cleaning method, after the liquid aspiration is completed, the first valve group 140 is switched to the second position, the pipette assembly 130 is in fluid communication with the first reagent kit 114, and the syringe 127 discharges the waste liquid into the first reagent kit 114.

### Gene Sequencer

The embodiments of the present disclosure further provide a gene sequencer. As shown in FIG. 1 and FIG. 2, a gene sequencer includes at least: a flow cell 115, a sample cartridge 113, a first reagent kit 114, a fluid driving assembly 109, and a first fluid loading module 108. Specifically, the flow cell 115 includes a plurality of lanes 107, inlets 142 and outlets 141. The sample cartridge 113 is used to store a sample, and the first reagent kit 114 is used to store a reagent required for sequencing. The fluid driving assembly 109 is used to generate a driving force to transport a sample or reagent in the fluid system. The first fluid loading module 108 is arranged on an outlet side of the flow cell 115, and is configured to be switchable at least between a first position for fluidly connecting the fluid driving assembly 109 to the sample cartridge 113 or the first reagent kit 114, a second position for fluidly connecting the fluid driving assembly 109 to the outlet 131 of the flow cell 115, and a third position for fluidly connecting the sample cartridge 113 or the first reagent kit 114 to the outlet 141 of the flow cell 115. More specifically, as shown in FIG. 2, the fluid driving assembly 109 further includes: a syringe 127 having a plurality of independent channels, and a plurality of reversing valves 128. The plurality of independent channels and the plurality of reversing valves 128 correspond to the plurality of lanes 107 of the flow cell 115 respectively, and the reversing valves 128 may be controlled individually or simultaneously.

More specifically, as shown in FIG. 1 and FIG. 2, the first fluid loading module 108 may include: a first valve group consisting of a plurality of first valves 140, a second valve group consisting of a plurality of second valves 137, and a first pipette assembly consisting of a plurality of first pipettes 130, where the plurality of first valves 140, the plurality of second valves 137 and the plurality of first pipettes 130 correspond to the plurality of lanes 107 of the flow cell 115 respectively. Each first valve 140 is connected to the fluid driving assembly 109, a corresponding first pipette 130 and a corresponding lane of the flow cell 115, and is configured to be switchable at least between a first position for fluidly connecting the fluid driving assembly 109 to the first pipette 130 and a second position for fluidly connecting the fluid driving assembly 109 to the outlet 141 of the corresponding lane 107 of the flow cell 115. Each second valve 137 is connected to the fluid driving assembly 109, a corresponding first valve 140 and a corresponding lane 107 of the flow cell 115, and is configured to be switchable at least between a first position for connecting the fluid driving assembly 109 to the corresponding first valve 140 and a second position for directly fluidly connecting the fluid driving assembly 109 to the outlet 141 of the corresponding lane 107 of the flow cell 115. In addition, the first fluid loading module 108 may further include: a plurality of groups of sensing assemblies 133 and 135. Each group of sensing assemblies 133 or 135 is connected between the fluid driving assembly 109 and the outlet 141 of a corresponding lane 107 of the flow cell 115, and is configured to monitor fluid characteristics.

Specifically, as shown in FIG. 1 and FIG. 2, the gene sequencer may further include a second fluid loading module 103. The second fluid loading module 103 is arranged on the inlet side of the flow cell 115, and is configured to be switchable at least between a first position for fluidly connecting the inlet 142 of the flow cell 115 to the sample cartridge 113 or the first reagent kit 114 and a second position for fluidly connecting the inlet 142 of the flow cell 115 to the fluid driving assembly 109. In addition, the gene sequencer may further include at least one second reagent kit 117 on the inlet side of the plurality of lanes 107 of the flow cell 115. The second fluid loading module 108 is arranged between the inlets 142 of the plurality of lanes 107 of the flow cell 115 and the at least one second reagent kit 117.

More specifically, as shown in FIG. 1 and FIG. 2, the second fluid loading module 103 may further include: a solenoid valve 146, a third valve group consisting of a plurality of third valves 148, a second pipette assembly consisting of a plurality of second pipettes 147, and a manifold assembly 144. The plurality of third valves 148 and the plurality of second pipettes 147 correspond to the plurality of lanes 107 of the flow cell 115 respectively. The plurality of second pipettes 147 are connected to at least one second reagent kit 117, and are in fluid communication with the plurality of lanes 107 of the flow cell 115 via the manifold assembly 144. As shown in FIG. 2, each third valve 148 has a first port and a second port, the first port is connected to the manifold assembly 144, and the second port is connected to a corresponding second pipette 147. Each third valve 148 is configured to be switchable between a first position for closing the fluid communication between the manifold assembly 144 and the corresponding second pipette 147 and a second position for opening the fluid communication between the manifold assembly 144 and the corresponding second pipette 147. Furthermore, the plurality of lanes 107 of the flow cell 115 are connected to the sample cartridge 113 or the first reagent kit 114 via the solenoid valve 146. It should be noted that, in the embodiment shown in FIG. 2, the plurality of first valves 140 in the first valve group, the plurality of second valves 137 in the second valve group and the plurality of third valves 148 in the third valve group may all be solenoid valves.

According to the embodiments of the present disclosure, the gene sequencer may further include: a fluid drive control assembly 101, a sample cartridge/reagent kit recovery module 116, a cleaning module 112, a cleaning liquid/pure water storage module 111, and a waste liquid storage module 110, etc. The fluid drive control assembly 101 is configured to control at least one of the fluid driving assembly 109, the first fluid loading module 108 or the second fluid loading module 103. The sample cartridge/reagent kit recovery module 116 is configured to recover the sample cartridge 113 and/or the first reagent kit 114. The cleaning module 112 is arranged between the fluid driving assembly 109 and the first fluid loading module 108, and is configured to perform cleaning of the plurality of lanes 107 of the flow cell 115. The cleaning liquid/pure water storage module 111 is in fluid communication with the cleaning module 112 and the second fluid loading module 103, and is configured to provide cleaning liquid and/or pure water when the cleaning of the plurality of lanes 107 of the flow cell 115 is performed. The waste liquid storage module 110 is in fluid communication with the cleaning module 112 and the fluid driving assembly 109, and is configured to recover and/or discharge waste liquid.

According to the embodiments of the present disclosure, as shown in FIG. 1 and FIG. 2, the gene sequencer may further include a first mechanical motion platform 104, a second mechanical motion platform 105, and a mechanical motion control assembly 102. Specifically, at least the flow cell 115 is placed on the first mechanical motion platform 104, and at least the sample cartridge 113, the first reagent kit 114 and the cleaning module 112 are placed on the second mechanical motion platform 105. The mechanical motion control assembly 102 is configured to control a multi-axis motion of the first mechanical motion platform 104 to selectively connect the flow cell 115 to the fluid transport pipeline network. The mechanical motion control assembly 102 is further configured to control a multi-axis motion of the second mechanical motion platform 105 to selectively connect one of the sample cartridge 113, the first reagent kit 114 or the cleaning module 112 to the fluid transport pipeline network. For example, at least one of the first mechanical motion platform 104 or the second mechanical motion platform 105 is a three-dimensional motion platform or a rotary motion platform.

### Biochemical Assay method

The embodiments of the present disclosure further provide a biochemical assay method, which may be combined with the aforementioned fluid system or gene sequencer to perform gene sequencing. The biochemical assay method provided by the embodiments of the present disclosure mainly includes the following steps: loading a sample to be detected into the lane of the flow cell from the outlet side of the flow cell; and loading the reagent containing a plurality of different reaction components into the lane of the flow cell from the inlet side or the outlet side of the flow cell, so as to perform a biochemical reaction between the sample and the reagent. The reaction components include at least one of a sample generation component or a sample analysis component. The biochemical reaction includes a generation step and/or a reaction step. In the generation step, a sample is generated in the lane of the flow cell; specifically, different sample generation components flow into the lane and a reaction condition in the lane is controlled, so as to generate the sample. The reaction step includes analyzing the sample in the lane; specifically, the sample analysis components flow into the lane, and the sample analysis components react with the sample to provide a related detectable signal. The detectable signal is preferably an optical signal. The biochemical reaction is a nucleic acid sequencing reaction, and the sample to be detected is a nucleic acid sequencing library.

Furthermore, the biochemical assay method provided by the embodiments of the present disclosure may further include: in a case of loading the reagent into the lane of the flow cell from a first reagent kit on the outlet side of the flow cell, discharging the reagent to the first reagent kit through the inlet of the flow cell after the biochemical reaction is performed; or in a case of loading the reagent into the lane of the flow cell from a second reagent kit on the inlet side of the flow cell, pushing the reagent back to the second reagent kit through the inlet of the flow cell after the biochemical reaction is performed. In addition, the biochemical assay method provided by the embodiments of the present disclosure may further include a cleaning step, in which the cleaning liquid/pure water is loaded into the lane of the flow cell from the outlet side of the flow cell, and the cleaning liquid/pure water is discharged to the first reagent kit, the second reagent kit or the waste liquid storage module through the inlet of the flow cell; or the cleaning liquid/pure water is loaded into the lane of the flow cell from the inlet side of the flow cell, and the cleaning liquid/pure water is pushed back to the second reagent kit or the waste liquid storage module through the inlet of the flow cell.

In the biochemical assay method provided by the embodiments of the present disclosure, the sample or reagent is reversely transported from the outlet to the inlet of the flow cell, and the reversely loaded sample or reagent may eventually be directly returned to the sample cartridge or the reagent kit. Furthermore, when cleaning of the lanes of the flow cell and the fluid transport pipeline network is performed, the waste liquid after cleaning may flow directly into the reagent kit. It is also possible to load the same or different samples into different lanes of the flow cell, so that the sequencing time may be reduced, and the flexibility of sample/reagent loading may be improved.

### Mechanical Motion Device for Fluid System

As shown in FIG. 1, the embodiments of the present disclosure further provide a mechanical motion device for a fluid system. The fluid system described here includes at least a sample cartridge 113, a reagent kit 114 and a flow cell 115, and may further include a cleaning module 112. The mechanical motion device may include a first mechanical motion platform 104, a second mechanical motion platform 105, and a mechanical motion control assembly 102. At least the flow cell 115 is placed on the first mechanical motion platform 104, and at least the sample cartridge 113 and the reagent kit 114 are placed on the second mechanical motion platform 105. In addition, the cleaning module 112 may also be placed on the second mechanical motion platform 105. The mechanical motion control assembly 102 is further configured to control a multi-axis motion of the second mechanical motion platform 105 to selectively connect one of the sample cartridge 113, the reagent kit 114 or the cleaning module 112 to the fluid transport pipeline network. Specifically, the mechanical motion control assembly 102 is configured to: control a multi-axis motion of the first mechanical motion platform 104 to connect the flow cell 115 to the fluid transport pipeline network, and control a multi-axis motion of the second mechanical motion platform 105 to selectively connect one of the sample cartridge 113 or the reagent kit 114 to the fluid transport pipeline network. For example, at least one of the first mechanical motion platform 104 or the second mechanical motion platform 105 may be a three-dimensional motion platform or a rotary motion platform. In this way, in the embodiments of the present disclosure, a sample loading system and a sequencer may be integrated by means of the control of the mechanical motion platform, and it is possible to load the same or different samples into different lanes of the sequencing chip on the gene sequencer, and it is also possible to load reagent, so that the sequencing steps may be reduced, and the sequencing efficiency may be improved.

### [Second Exemplary Embodiment]

It should be noted that the second exemplary embodiment and various exemplary embodiments and optional embodiments to be described below are all based on the first exemplary embodiment described above, and an addition, reduction or replacement of components/parts/steps is based on components/parts/steps of the system/device/method, etc. mentioned in the first exemplary embodiment. For the purpose of clarity, only differences of the second exemplary embodiment and various exemplary embodiments to be described below from the first exemplary embodiment will be described below. Accordingly, the same or similar components/parts/steps are denoted by the same or similar reference numerals.

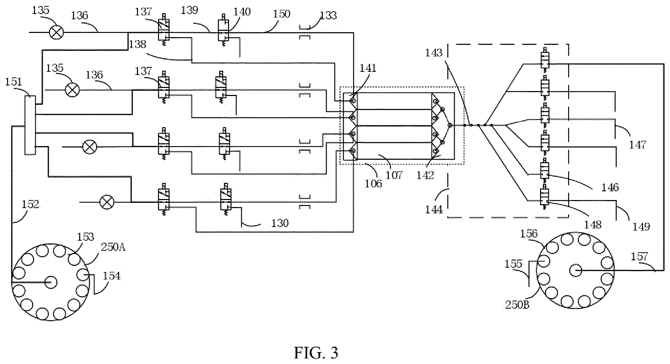

FIG. 3 shows a schematic diagram of a fluid system according to the second exemplary embodiment of the present disclosure. Referring to FIG. 3, different from the first exemplary embodiment, in the second exemplary embodiment, a first rotary valve 250A and a second rotary valve 250B are respectively added to the outlet side and the inlet side of the plurality of lanes of the flow cell. The first rotary valve 250A is configured to selectively connect one of the sample cartridge or the first reagent kit to the fluid transport pipeline network of the fluid system, and the second rotary valve 250B is configured to selectively connect one of the sample cartridge, the first reagent kit or the second reagent kit to the fluid transport pipeline network of the fluid system. For example, a sample from the sample cartridge or a reagent from the first reagent kit may be loaded to the outlet side of the lanes of the flow cell through an interface 153 or 154 of the first rotary valve 250A. For example, a reagent from the first reagent kit may be loaded to the outlet side of the lanes of the flow cell through the interface 153 of the first rotary valve 250A, and a reagent from the second reagent kit may be loaded to the inlet side of the lanes of the flow cell through an interface 156 of the second rotary valve 250B. In addition, the pipeline 157 in the second rotary valve 250B may also be directly connected to the common pipeline 143 in the manifold assembly 144 without passing through the third valve group 148.

### [Third Exemplary Embodiment]

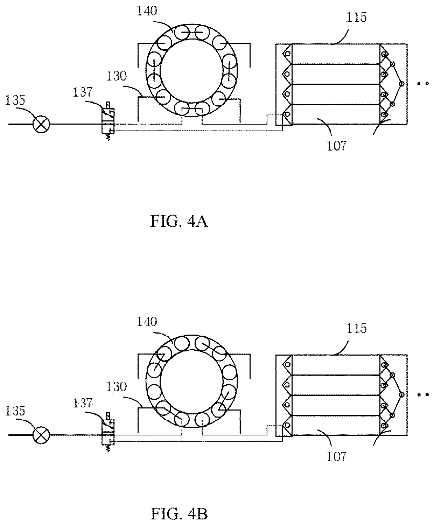

Different from the first exemplary embodiment of the present disclosure, in the fluid system provided by the third exemplary embodiment of the present disclosure, the third valves in the third valve group are multi-way rotary valves instead of solenoid valves. FIG. 4A and FIG. 4B show partial schematic diagrams of the fluid system according to the third exemplary embodiment of the present disclosure, in which states of a multi-way rotary valve in a first position and a second position are shown respectively. In the exemplary embodiment shown in FIG. 4A and FIG. 4B, a 2-position 12-way rotary valve is taken as an example for the multi-way rotary valve, but a 2-position 8-way rotary valve or a multi-position multi-way rotary valve may also be used. When the rotary valve 140 is in the first position as shown in FIG. 4A, the solenoid valve in the second valve group 137 is in fluid communication with the lane 107 of the flow cell through the rotary valve 140. When the rotary valve 140 is in the second position as shown in FIG. 4B, the solenoid valve in the second valve group 137 is in fluid communication with the first pipette assembly 130 through the rotary valve 140.

### [First Optional Embodiment]

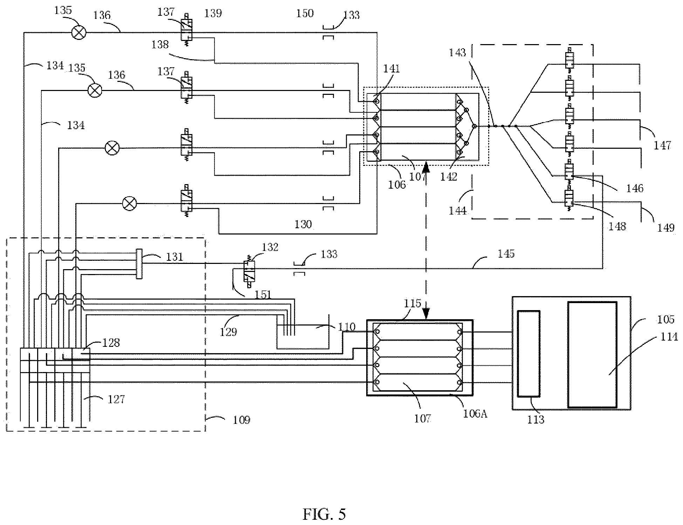

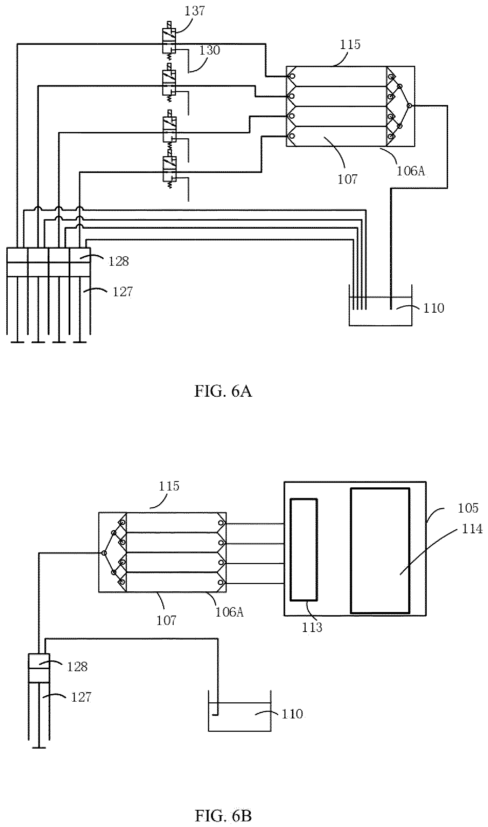

In the fluid system described in the first exemplary embodiment of the present disclosure, the flow cell is placed on the chip-carrying platform module, and the sample/reagent loading and sequencing processes are directly performed without moving the flow cell. In contrast, a first optional embodiment in FIG. 5, FIG. 6A and FIG. 6B shows a dual-platform fluid system having both a sequencing platform and a loading platform. FIG. 5 shows a schematic diagram of a fluid system according to the first optional embodiment of the present disclosure; and FIG. 6A and FIG. 6B show partial schematic diagrams of the fluid system according to the first optional embodiment of the present disclosure, in which a flow path of the loading platform 106A is shown.

The dual-platform fluid system shown in FIG. 5 includes a non-integrated flow cell. For the non-integrated flow cell, as shown in FIG. 5, the flow cell 115 is firstly placed on the loading platform 106A, and the sample cartridge 113 and the first reagent kit 114 may be connected to the interface of the flow cell 115 in sequence through the control of the second mechanical motion platform 105. The hole positions of the sample cartridge 113 or the first reagent kit 114 correspond to the lanes 107 in the flow cell 115 respectively. The fluid driving assembly 109 provides a driving force to directly aspirate the sample in the sample cartridge 113 or the reagent in the reagent kit 114 for loading, and the excess reagent is directly discharged into the waste liquid storage module 110. After the reaction is completed, the flow cell 115 may be transferred to the chip-carrying platform module (which is the sequencing platform in this embodiment) 106 by an automatic transfer device for a subsequent biochemical reaction. The automatic transfer device may be a machine hand or a mechanical claw.

The dual-platform fluid system provided in this embodiment may also include an integrated flow cell. For the integrated flow cell, as shown in FIG. 6A and FIG. 6B, a plurality of lane interfaces at one end of the flow cell 115 converge into a port (e.g., a right port in FIG. 6A and a left port in FIG. 6B), and samples or reagents may be loaded using positive pressure or negative pressure. The integrated flow cell shown in FIG. 6A may be loaded with samples or reagents by means of positive pressure. Specifically, the sample cartridge or the first reagent kit may be in fluid communication with the pipette assembly 130 in sequence through the control of the second mechanical motion platform 105, and the pipette assembly 130 corresponds to the lanes 107 in the flow cell 115 respectively. The solenoid valve 137 is switched to be in fluid communication with the pipette assembly 130 and the fluid driving assembly (including the syringe 127 and the reversing valve 128), and the fluid driving assembly provides a negative pressure driving force to aspirate samples or reagents to between the solenoid valve 137 and the fluid driving assembly. After the fluid aspiration is completed, the solenoid valve 137 is switched so that the flow cell 115 is in fluid communication with the fluid driving assembly. The fluid driving assembly provides a positive pressure driving force to directly push the sample or reagent into the lane 107 of the flow cell 115 for biochemical reaction, and the excess reagent enters the waste liquid storage module 110. The integrated flow cell shown in FIG. 6B may be loaded with samples or reagents by means of negative pressure. Specifically, the sample cartridge 113 or the first reagent kit 114 is in direct fluid communication with the flow cell 115 located on the loading platform 106A, and the other end of the flow cell 115 is in fluid communication with the fluid driving assembly (including the syringe 127 and the reversing valve 128). The fluid driving assembly provides a negative pressure driving force to directly aspirate samples or reagents into the lane 107 of the flow cell 115 for biochemical reaction. In addition, the reversing valve 128 in the fluid driving assembly may be switched so that the excess reagent enters the waste liquid storage module 110.

### [Second Optional Embodiment]

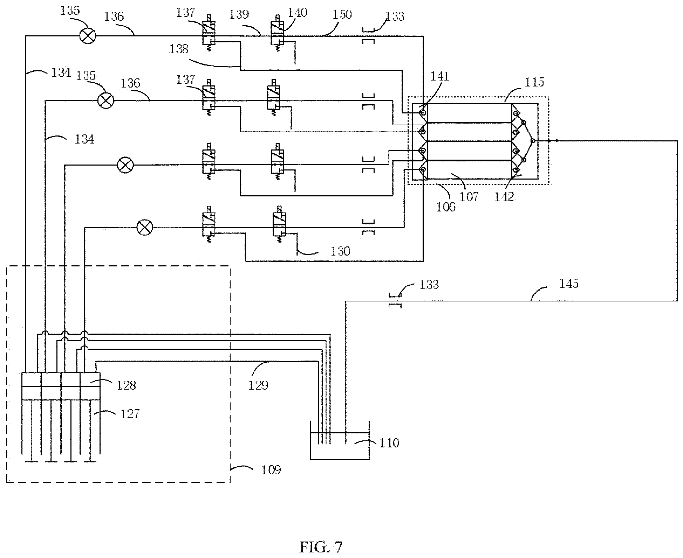

In the fluid system described in the first exemplary embodiment of the present disclosure, the first fluid loading module 108 is arranged on the outlet side of the flow cell 115, while the second fluid loading module 103 is arranged on the inlet side. Different from that, in the second optional embodiment, only the first fluid loading module 108 is provided on the outlet side of the flow cell, while no fluid loading module is provided on the inlet side. FIG. 7 shows a schematic diagram of a fluid system according to the second optional embodiment of the present disclosure.

Referring to FIG. 7, since no fluid control device is provided on the inlet side of the flow cell 115, after the sample or reagent completes the biochemical reaction in the lane 107 of the flow cell 115, the excess reagent directly enters the waste liquid storage module 110 through a lane integrated port on the inlet side of the flow cell 115.

Those skilled in the art may understand that the embodiments described above are exemplary and may be improved by those skilled in the art. The structures described in various embodiments may be freely combined without causing conflicts in structure or principle.

The present disclosure has been described with reference to the accompanying drawings. However, the embodiments disclosed in the accompanying drawings are intended to exemplify the implementation methods of the present disclosure and should not be construed as limiting the present disclosure.

Some embodiments of the present disclosure have been shown and described. However, those skilled in the art may understand that changes may be made to these embodiments without departing from the principles and spirit of the present disclosure, and the scope of the present disclosure is defined by the claims and their equivalents.

## Claims

1. A fluid transport method, comprising:
step a) of aspirating a first fluid from a fluid cartridge by using a pump valve assembly, so that the first fluid is input from an outlet of a flow cell (115) into a lane of the flow cell, and the first fluid output from an inlet of the flow cell is returned to the fluid cartridge; and/or
step b) of aspirating a second fluid from a fluid cartridge by using a pump valve assembly, so that the second fluid is input from the inlet of the flow cell into the lane of the flow cell, and the second fluid output from the outlet of the flow cell is returned to the fluid cartridge.

2. The fluid transport method according to claim 1, wherein the pump valve assembly comprises a fluid driving assembly (109) configured to provide a positive pressure driving force or a negative pressure driving force in the step a) and the step b); and
wherein the step a) comprises:
a positive pressure driving step of generating the positive pressure driving force on an outlet side of the flow cell so that the first fluid is input into the lane from the outlet; or
a negative pressure driving step of generating the negative pressure driving force on an inlet side of the flow cell so that the first fluid is input into the lane from the outlet.

3. The fluid transport method according to claim 2, wherein the pump valve assembly further comprises a first fluid loading module (108) configured to be switchable at least between a first position for fluidly connecting the fluid driving assembly to the fluid cartridge and a second position for fluidly connecting the fluid driving assembly to the outlet of the flow cell; and
wherein the step a) comprises: firstly switching the first fluid loading module to the first position so that the negative pressure driving force is generated to aspirate the first fluid from the fluid cartridge, and then switching the first fluid loading module to the second position so that the positive pressure driving step is performed on the outlet side of the flow cell.

4. The fluid transport method according to claim 3, wherein the pump valve assembly further comprises a second fluid loading module (103) configured to be switchable at least between a first position for fluidly connecting the inlet of the flow cell to the fluid cartridge and a second position for fluidly connecting the inlet of the flow cell to the fluid driving assembly;
wherein the first fluid loading module is further configured to be switchable between the first position, the second position, and a third position for fluidly connecting the fluid cartridge to the outlet of the flow cell; and
wherein the step a) comprises:
firstly switching the first fluid loading module to the first position so that the negative pressure driving force is generated to aspirate the first fluid from the fluid cartridge, and then switching the first fluid loading module to the second position and switching the second fluid loading module to the first position so that the positive pressure driving step is performed on the outlet side of the flow cell; or
switching the first fluid loading module to the third position and switching the second fluid loading module to the second position so that the negative pressure driving step is performed on the inlet side of the flow cell.

5. The fluid transport method according to claim 4, wherein the fluid cartridge comprises a sample cartridge (113) and a first reagent kit (114) arranged in parallel, and the fluid transport method further comprises:
selectively performing a fluid transport of a sample in the sample cartridge or a fluid transport of a reagent in the first reagent kit.

6. The fluid transport method according to claim 5, wherein a second reagent kit (117) in fluid communication with the second fluid loading module is provided, and the second fluid loading module is further configured to be switchable between the first position, the second position, and a third position for fluidly connecting the second reagent kit to the inlet of the flow cell; and
wherein the step b) comprises: switching the first fluid loading module to the second position and switching the second fluid loading module to the third position, so that the negative pressure driving force is generated on the outlet side of the flow cell to input a reagent in the second reagent kit into the lane of the flow cell from the inlet.

7. The fluid transport method according to claim 6, wherein a fluid drive control assembly (101) is provided, and the fluid transport method further comprises:
controlling at least one of the fluid driving assembly, the first fluid loading module or the second fluid loading module through the fluid drive control assembly.

8. The fluid transport method according to claim 6, wherein a sample cartridge/reagent kit recovery module (116) is provided, and the fluid transport method further comprises:
recovering the sample cartridge and/or the first reagent kit through the sample cartridge/reagent kit recovery module.

9. The fluid transport method according to claim 6, wherein the fluid cartridge further comprises a cleaning module (112), a cleaning liquid/pure water storage module (111) in fluid communication with the cleaning module, and a waste liquid storage module (110) in fluid communication with the cleaning module, and the fluid transport method further comprises:
performing cleaning of the lane of the flow cell by using the cleaning module, the cleaning liquid/pure water storage module and the waste liquid storage module.

10. The fluid transport method according to claim 9, wherein the cleaning module is arranged in parallel with the sample cartridge and the first reagent kit, and the fluid transport method further comprises:
selectively performing the fluid transport of the sample in the sample cartridge, the fluid transport of the reagent in the first reagent kit, or the cleaning of the lane of the flow cell; and
performing the cleaning of the lane of the flow cell through the positive pressure driving step or the negative pressure driving step in the step a) or through the step b) when the cleaning of the lane of the flow cell is selectively performed.

11. The fluid transport method according to claim 9, wherein a first mechanical motion platform (104), a second mechanical motion platform (105) and a mechanical motion control assembly (102) are provided, at least the flow cell is placed on the first mechanical motion platform, and at least the sample cartridge, the first reagent kit and the cleaning module are placed on the second mechanical motion platform; and
wherein the fluid transport method further comprises:
controlling a multi-axis motion of the first mechanical motion platform through the mechanical motion control assembly to connect the flow cell to a fluid transport pipeline network; and
controlling a multi-axis motion of the second mechanical motion platform through the mechanical motion control assembly to selectively connect one of the sample cartridge, the first reagent kit or the cleaning module to the fluid transport pipeline network.

12. The fluid transport method according to claim 6, wherein the first fluid is different from the second fluid, the first fluid is selected from a sample, a reagent, a cleaning liquid or pure water, and the second fluid is selected from a reagent, a cleaning liquid or pure water.

13. A fluid system, comprising:
a flow cell (115) comprising a plurality of lanes (107), inlets (142) and outlets (141);
a sample cartridge (113) and a first reagent kit (114), wherein the sample cartridge is configured to store a sample, and the first reagent kit is configured to store a reagent for sequencing;
a fluid driving assembly (109) configured to generate a driving force to transport the sample or the reagent in the fluid system; and
a first fluid loading module (108) on an outlet side of the flow cell, wherein the first fluid loading module is configured to be switchable at least between a first position for fluidly connecting the fluid driving assembly to a fluid cartridge, a second position for fluidly connecting the fluid driving assembly to the outlet of the flow cell, and a third position for fluidly connecting the fluid cartridge to the outlet of the flow cell.

14. The fluid system according to claim 13, wherein the first fluid loading module comprises:
a first valve group (140) consisting of a plurality of first valves, a second valve group (137) consisting of a plurality of second valves, and a first pipette assembly (130) consisting of a plurality of first pipettes,
wherein the plurality of first valves, the plurality of second valves and the plurality of first pipettes correspond to the plurality of lanes of the flow cell respectively;
wherein each first valve is connected to the fluid driving assembly, a corresponding first pipette and a corresponding lane of the flow cell, and each first valve is configured to be switchable at least between a first position for fluidly connecting the fluid driving assembly to the first pipette and a second position for fluidly connecting the fluid driving assembly to an outlet of the corresponding lane of the flow cell; and
wherein each second valve is connected to the fluid driving assembly, a corresponding first valve and a corresponding lane of the flow cell, and each second valve is configured to be switchable at least between a first position for connecting the fluid driving assembly to the corresponding first valve and a second position for directly fluidly connecting the fluid driving assembly to an outlet of the corresponding lane of the flow cell.

15. The fluid system according to claim 13, wherein the first fluid loading module further comprises:
a plurality of groups of sensing assemblies (133, 135), wherein each group of sensing assemblies is connected between the fluid driving assembly and an outlet of a corresponding lane of the flow cell, and each group of sensing assemblies is configured to monitor fluid characteristics.

16. The fluid system according to claim 14, further comprising:
a second fluid loading module (103) on an inlet side of the flow cell, wherein the second fluid loading module is configured to be switchable at least between a first position for fluidly connecting the inlet of the flow cell to the fluid cartridge and a second position for fluidly connecting the inlet of the flow cell to the fluid driving assembly.

17. The fluid system according to claim 16, further comprising:
at least one second reagent kit (117) on an inlet side of the plurality of lanes of the flow cell,
wherein the second fluid loading module is arranged between the inlets of the plurality of lanes of the flow cell and the at least one second reagent kit.

18. The fluid system according to claim 17, wherein the second fluid loading module further comprises:
a solenoid valve (146), a third valve group (148) consisting of a plurality of third valves, a second pipette assembly (147) consisting of a plurality of second pipettes, and a manifold assembly (144),
wherein the plurality of third valves and the plurality of second pipettes correspond to the plurality of lanes of the flow cell respectively, and the plurality of second pipettes are connected to the at least one second reagent kit and are in fluid communication with the plurality of lanes of the flow cell via the manifold assembly;
wherein each third valve has a first port connected to the manifold assembly and a second port connected to a corresponding second pipette, and each third valve is configured to be switchable between a first position for closing fluid communication between the manifold assembly and the corresponding second pipette and a second position for opening the fluid communication between the manifold assembly and the corresponding second pipette; and
wherein the plurality of lanes of the flow cell are connected to the sample cartridge or the first reagent kit via the solenoid valve (146).

19. The fluid system according to claim 18, further comprising:
a fluid drive control assembly (101) configured to control at least one of the fluid driving assembly, the first fluid loading module or the second fluid loading module.

20. The fluid system according to claim 18, further comprising:
a sample cartridge/reagent kit recovery module (116) configured to recover the sample cartridge and/or the first reagent kit.

21. The fluid system according to claim 18, further comprising:
a cleaning module (112) between the fluid driving assembly and the first fluid loading module, wherein the cleaning module is configured to perform cleaning of the plurality of lanes of the flow cell;
a cleaning liquid/pure water storage module (111) in fluid communication with the cleaning module and the second fluid loading module, wherein the cleaning liquid/pure water storage module is configured to provide cleaning liquid and/or pure water when the cleaning of the plurality of lanes of the flow cell is performed; and
a waste liquid storage module (110) in fluid communication with the cleaning module and the fluid driving assembly, wherein the waste liquid storage module is configured to recover and/or discharge waste liquid.

22. The fluid system according to claim 21, wherein the fluid driving assembly comprises: a syringe (127) having a plurality of independent channels, and a plurality of reversing valves (128);
wherein the plurality of independent channels and the plurality of reversing valves correspond to the plurality of lanes of the flow cell respectively; and
wherein the plurality of reversing valves are controllable individually or simultaneously.

23. The fluid system according to claim 22, further comprising:
a first mechanical motion platform (104), a second mechanical motion platform (105), and a mechanical motion control assembly (102),
wherein at least the flow cell is placed on the first mechanical motion platform, and at least the sample cartridge, the first reagent kit and the cleaning module are placed on the second mechanical motion platform;
wherein the mechanical motion control assembly is configured to control a multi-axis motion of the first mechanical motion platform to selectively connect the flow cell to a fluid transport pipeline network of the fluid system; and
wherein the mechanical motion control assembly is further configured to control a multi-axis motion of the second mechanical motion platform to selectively connect one of the sample cartridge, the first reagent kit or the cleaning module to the fluid transport pipeline network of the fluid system.

24. The fluid system according to claim 23, wherein at least one of the first mechanical motion platform or the second mechanical motion platform is a three-dimensional motion platform or a rotary motion platform.

25. The fluid system according to claim 23, wherein the plurality of first valves in the first valve group, the plurality of second valves in the second valve group and the plurality of third valves in the third valve group are solenoid valves.

26. The fluid system according to claim 23, wherein the plurality of first valves in the first valve group are multi-way rotary valves, and the plurality of second valves in the second valve group and the plurality of third valves in the third valve group are solenoid valves.

27. The fluid system according to claim 26, wherein the multi-way rotary valve is a 2-position 8-way rotary valve or a 2-position 12-way rotary valve.

28. The fluid system according to claim 23, further comprising:
a first rotary valve (250A) on the outlet side of the plurality of lanes of the flow cell, wherein the first rotary value is configured to selectively connect one of the sample cartridge or the first reagent kit to the fluid transport pipeline network of the fluid system; and
a second rotary valve (250B) on the inlet side of the plurality of lanes of the flow cell, wherein the second rotary valve is configured to selectively connect one of the sample cartridge, the first reagent kit or the second reagent kit to the fluid transport pipeline network of the fluid system.

29. The fluid system according to claim 16, further comprising:
a sequencing platform (106) between the first fluid loading module and the second fluid loading module;
a loading platform (106A) between the sample cartridge and the fluid driving assembly and between the first reagent kit and the fluid driving assembly; and
an automatic transfer device configured to automatically transfer the flow cell between the sequencing platform and the loading platform.

30. A gene sequencer, comprising:
a flow cell (115) comprising a plurality of lanes (107), inlets (142) and outlets (141);
a sample cartridge (113) and a first reagent kit (114), wherein the sample cartridge is configured to store a sample, and the first reagent kit is configured to store a reagent for sequencing;
a fluid driving assembly (109) configured to generate a driving force to transport the sample or the reagent in a fluid system; and
a first fluid loading module (108) on an outlet side of the flow cell, wherein the first fluid loading module is configured to be switchable at least between a first position for fluidly connecting the fluid driving assembly to the sample cartridge or the first reagent kit, a second position for fluidly connecting the fluid driving assembly to the outlet of the flow cell, and a third position for fluidly connecting the sample cartridge or the first reagent kit to the outlet of the flow cell.

31. The gene sequencer according to claim 30, wherein the first fluid loading module comprises:
a first valve group (140) consisting of a plurality of first valves, a second valve group (137) consisting of a plurality of second valves, and a first pipette assembly (130) consisting of a plurality of first pipettes,
wherein the plurality of first valves, the plurality of second valves and the plurality of first pipettes correspond to the plurality of lanes of the flow cell respectively;
wherein each first valve is connected to the fluid driving assembly, a corresponding first pipette and a corresponding lane of the flow cell, and each first valve is configured to be switchable at least between a first position for fluidly connecting the fluid driving assembly to the first pipette and a second position for fluidly connecting the fluid driving assembly to an outlet of the corresponding lane of the flow cell; and
wherein each second valve is connected to the fluid driving assembly, a corresponding first valve and a corresponding lane of the flow cell, and each second valve is configured to be switchable at least between a first position for connecting the fluid driving assembly to the corresponding first valve and a second position for directly fluidly connecting the fluid driving assembly to an outlet of the corresponding lane of the flow cell.

32. The gene sequencer according to claim 30, wherein the first fluid loading module further comprises:
a plurality of groups of sensing assemblies (133, 135), wherein each group of sensing assemblies is connected between the fluid driving assembly and an outlet of a corresponding lane of the flow cell, and each group of sensing assemblies is configured to monitor fluid characteristics.

33. The gene sequencer according to claim 31, further comprising:
a second fluid loading module (103) on an inlet side of the flow cell, wherein the second fluid loading module is configured to be switchable at least between a first position for fluidly connecting the inlet of the flow cell to the sample cartridge or the first reagent kit and a second position for fluidly connecting the inlet of the flow cell to the fluid driving assembly.

34. The gene sequencer according to claim 33, further comprising:
at least one second reagent kit (117) on an inlet side of the plurality of lanes of the flow cell,
wherein the second fluid loading module is arranged between the inlets of the plurality of lanes of the flow cell and the at least one second reagent kit.

35. The gene sequencer according to claim 34, wherein the second fluid loading module further comprises:
a solenoid valve (146), a third valve group (148) consisting of a plurality of third valves, a second pipette assembly (147) consisting of a plurality of second pipettes, and a manifold assembly (144),
wherein the plurality of third valves and the plurality of second pipettes correspond to the plurality of lanes of the flow cell respectively, and the plurality of second pipettes are connected to the at least one second reagent kit and are in fluid communication with the plurality of lanes of the flow cell via the manifold assembly;
wherein each third valve has a first port connected to the manifold assembly and a second port connected to a corresponding second pipette, and each third valve is configured to be switchable between a first position for closing fluid communication between the manifold assembly and the corresponding second pipette and a second position for opening the fluid communication between the manifold assembly and the corresponding second pipette; and
wherein the plurality of lanes of the flow cell are connected to the sample cartridge or the first reagent kit via the solenoid valve (146).

36. The gene sequencer according to claim 35, further comprising:
a fluid drive control assembly (101) configured to control at least one of the fluid driving assembly, the first fluid loading module or the second fluid loading module.

37. The gene sequencer according to claim 35, further comprising:
a sample cartridge/reagent kit recovery module (116) configured to recover the sample cartridge and/or the first reagent kit.

38. The gene sequencer according to claim 35, further comprising:
a cleaning module (112) between the fluid driving assembly and the first fluid loading module, wherein the cleaning module is configured to perform cleaning of the plurality of lanes of the flow cell;
a cleaning liquid/pure water storage module (111) in fluid communication with the cleaning module and the second fluid loading module, wherein the cleaning liquid/pure water storage module is configured to provide cleaning liquid and/or pure water when the cleaning of the plurality of lanes of the flow cell is performed; and
a waste liquid storage module (110) in fluid communication with the cleaning module and the fluid driving assembly, wherein the waste liquid storage module is configured to recover and/or discharge waste liquid.

39. The gene sequencer according to claim 38, wherein the fluid driving assembly comprises: a syringe (127) having a plurality of independent channels, and a plurality of reversing valves (128);
wherein the plurality of independent channels and the plurality of reversing valves correspond to the plurality of lanes of the flow cell respectively; and
wherein the plurality of reversing valves are controllable individually or simultaneously.

40. The gene sequencer according to claim 39, further comprising:
a first mechanical motion platform (104), a second mechanical motion platform (105), and a mechanical motion control assembly (102),
wherein at least the flow cell is placed on the first mechanical motion platform, and at least the sample cartridge, the first reagent kit and the cleaning module are placed on the second mechanical motion platform;
wherein the mechanical motion control assembly is configured to control a multi-axis motion of the first mechanical motion platform to selectively connect the flow cell to a fluid transport pipeline network of the fluid system; and
wherein the mechanical motion control assembly is further configured to control a multi-axis motion of the second mechanical motion platform to selectively connect one of the sample cartridge, the first reagent kit or the cleaning module to the fluid transport pipeline network of the fluid system.

41. The gene sequencer according to claim 40, wherein at least one of the first mechanical motion platform or the second mechanical motion platform is a three-dimensional motion platform or a rotary motion platform.

42. The gene sequencer according to claim 40, wherein the plurality of first valves in the first valve group, the plurality of second valves in the second valve group and the plurality of third valves in the third valve group are solenoid valves.

43. The gene sequencer according to claim 40, wherein the plurality of first valves in the first valve group are multi-way rotary valves, and the plurality of second valves in the second valve group and the plurality of third valves in the third valve group are solenoid valves.

44. The gene sequencer according to claim 43, wherein the multi-way rotary valve is a 2-position 8-way rotary valve or a 2-position 12-way rotary valve.

45. The gene sequencer according to claim 40, further comprising:
a first rotary valve (250A) on the outlet side of the plurality of lanes of the flow cell, wherein the first rotary value is configured to selectively connect one of the sample cartridge or the first reagent kit to the fluid transport pipeline network of the fluid system; and
a second rotary valve (250B) on the inlet side of the plurality of lanes of the flow cell, wherein the second rotary valve is configured to selectively connect one of the sample cartridge, the first reagent kit or the second reagent kit to the fluid transport pipeline network of the fluid system.

46. The gene sequencer according to claim 33, further comprising:
a sequencing platform between the first fluid loading module and the second fluid loading module;
a loading platform between the sample cartridge and the fluid driving assembly and between the first reagent kit and the fluid driving assembly; and
an automatic transfer device configured to automatically transfer the flow cell between the sequencing platform and the loading platform.

47. A biochemical assay method, comprising:
loading a sample to be detected into a lane of a flow cell from an outlet side of the flow cell; and
loading a reagent containing a plurality of different reaction components into the lane of the flow cell from an inlet side or the outlet side of the flow cell, so as to perform a biochemical reaction between the sample and the reagent,
wherein the reaction component comprises at least one of a sample generation component or a sample analysis component;
wherein the biochemical reaction comprises: generating a sample in the lane of the flow cell, by allowing different sample generation components to flow into the lane and controlling a reaction condition in the lane; and
wherein the biochemical reaction further comprises: analyzing the sample in the lane, by allowing the sample analysis component to flow into the lane and react with the sample to provide a related detectable signal.

48. The biochemical assay method according to claim 47, wherein the biochemical reaction is a nucleic acid sequencing reaction, and the sample to be detected is a nucleic acid sequencing library.

49. The biochemical assay method according to claim 47, wherein the detectable signal is an optical signal.

50. The biochemical assay method according to claim 47, further comprising:
in a case of loading the reagent into the lane of the flow cell from a first reagent kit on the outlet side of the flow cell, discharging the reagent to the first reagent kit via an inlet of the flow cell after performing the biochemical reaction; or
in a case of loading the reagent into the lane of the flow cell from a second reagent kit on the inlet side of the flow cell, pushing the reagent back to the second reagent kit via the inlet of the flow cell after performing the biochemical reaction.

51. The biochemical assay method according to claim 50, further comprising:
loading cleaning liquid/pure water into the lane of the flow cell from the outlet side of the flow cell, and discharging the cleaning liquid/pure water to the first reagent kit, the second reagent kit or a waste liquid storage module via the inlet of the flow cell; or
loading the cleaning liquid/pure water into the lane of the flow cell from the inlet side of the flow cell, and pushing the cleaning liquid/pure water back to the second reagent kit or the waste liquid storage module via the inlet of the flow cell.

52. A mechanical motion device for a fluid system, wherein the fluid system comprises at least a sample cartridge (113), a reagent kit (114) and a flow cell (115); the mechanical motion device comprising:
a first mechanical motion platform (104), wherein the flow cell is placed on the first mechanical motion platform;
a second mechanical motion platform (105), wherein the sample cartridge and the reagent kit are placed on the second mechanical motion platform; and
a mechanical motion control assembly (102) configured to: control a multi-axis motion of the first mechanical motion platform to connect the flow cell to a fluid transport pipeline network of the fluid system, and control a multi-axis motion of the second mechanical motion platform to selectively connect one of the sample cartridge or the reagent kit to the fluid transport pipeline network.

53. The mechanical motion device according to claim 52, wherein the fluid system further comprises a cleaning module (112), and the cleaning module is placed on the second mechanical motion platform; and
wherein the mechanical motion control assembly is further configured to control the multi-axis motion of the second mechanical motion platform to selectively connect one of the sample cartridge, the reagent kit or the cleaning module to the fluid transport pipeline network.

54. The mechanical motion device according to claim 52, wherein at least one of the first mechanical motion platform or the second mechanical motion platform is a three-dimensional motion platform or a rotary motion platform.
